# EUROPEAN PATENT SPECIFICATION

(11) **EP 2 146 731 B1**
(45) Date of publication and mention of the grant of the patent: **16.01.2019**
(21) Application number: 08737019.3
(22) Date of filing: 17.04.2008
(51) Int. Cl.: A61K 36/185, A61P 35/00

(54) **CANNABINOID-CONTAINING PLANT EXTRACTS FOR THE TREATMENT OF PROSTATE CANCER**
CANNABINOID-ENTHALTENDEN PFLANZENEXTRAKTEN ZUR BEHANDLUNG VON PROSTATAKREBS
EXTRAITS VÉGÉTAUX CONTENANT DES CANNABINOÏDES POUR LE TRAITEMENT DU CANCER DE LA PROSTATE

(30) Priority: 19.04.2007 GB 0707610
(43) Date of publication of application: 27.01.2010
(73) Proprietor: GW Pharma Limited, Histon Cambridge CB24 9BZ (GB)
(72) Inventor: DI MARZO, Vincenzo, I-80078 Pozzuoli (IT); DE PETROCELLIS, Luciano, I-80078 Pozzuoli (IT); SCHIANO MORIELLO, Aniello, I-80078 Pozzuoli (IT)
(74) Representative: HGF Limited
(86) International application number: PCT/GB2008/001359
(87) International publication number: WO 2008/129258

(56) References cited:
- WO-A-02/064109
- WO-A-02/069993
- WO-A-2005/120478
- WO-A2-2007/024496
- US-A1- 2003 021 752
- US-B1- 6 949 582
- LONG LEONORA E ET AL: "The pharmacological actions of cannabidiol" DRUGS OF THE FUTURE, vol. 30, no. 7, July 2005 (2005-07), pages 747-753, XP002489015 ISSN: 0377-8282
- NURMIKKO TURO J ET AL: "A MULTI-CENTRE, DOUBLE-BLIND, RANDOMIZED, PLACEBO-CONTROLLED TRIAL OF ORO-MUCOSAL CANNABIS-BASED MEDICINE IN THE TREATMENT OF NEUROPATHIC PAIN CHARACTERIZED BY ALLODYNIA" NEUROLOGY, LIPPINCOTT WILLIAMS & WILKINS, PHILADELPHIA, US, vol. 64, no. 6 SUPPL. 1, 19 April 2005 (2005-04-19), page A374, XP009076274 ISSN: 0028-3878
- SMITH PAUL F: "GW-1000. GW PHARMACEUTICALS" CURRENT OPINION IN INVESTIGATIONAL DRUGS, PHARMAPRESS, US, vol. 5, no. 7, 1 July 2004 (2004-07-01), pages 748-754, XP009076281 ISSN: 1472-4472
- LIGRESTI A ET AL: "Antitumor activity of plant cannabinoids with emphasis on the effect of cannabidiol on human breast carcinoma", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 318, no. 3, 1 January 2006 (2006-01-01), pages 1375-1387, XP002540820, ISSN: 0022-3565, DOI: 10.1124/JPET.106.105247
- M. M. Caffarel: " 9-Tetrahydrocannabinol Inhibits Cell Cycle Progression in Human Breast Cancer Cells through Cdc2 Regulation", Cancer research, vol. 66, no. 13, 1 July 2006 (2006-07-01), pages 6615-6621, XP55048739, ISSN: 0008-5472, DOI: 10.1158/0008-5472.CAN-05-4566
- PERNA F ET AL: "Cannabinoids induces apoptosis in human colon cancer cells via CB2 receptor activation", DIGESTIVE AND LIVER DISEASE, W.B. SAUNDERS, GB, vol. 38, 1 April 2006 (2006-04-01), pages S107-S108, XP025948651, ISSN: 1590-8658, DOI: 10.1016/S1590-8658(06)80287-2 [retrieved on 2006-04-01]

## Description

The present invention relates to one or more cannabinoids, which are TRPM8 antagonists, selected from the group consisting of cannabidiol (CBD); cannabigerol (CBG); and cannabidiolic acid (CBDA) for use in the prevention or treatment of cancer of the prostate, where TRPM8 activity is essential for the cancers survival.

### BACKGROUND TO THE INVENTION

Transient receptor potential (TRP) channels are known to be at the forefront of mammals sensory systems, and have been found to be involved in the response to temperature, touch, pain, osmolarity, pheromones, taste and other stimuli. It is thought that the role of TRP channels is far broader than simple sensory transduction as they are able to respond to many stimuli from both inside and outside of the cell.

Mammals are able to detect temperature with specialised neurons in their peripheral nervous system. These neurones are a subset of TRP channels: vanilloid-type channels (TRPV). Four different TRPV channels (TRPV1-4) have been identified and are implicated in heat sensing. These are temperature sensitive ion channels and are critical contributors to normal pain and temperature sensation. As such they are useful targets for the relief of pain.

A different subset, the TRPM channels (melastatin-type), in particular the TRPM8 channel, is implicated in sensing cold temperatures of less than 25°C. The combined range of temperatures that these channels are able to detect covers the majority of the relevant 'normal range' temperatures that are sensed by most mammals. Externally applied agents such as menthol, eucalyptol and icilin are able to activate the TRPM8 channels.

Up-regulation of activity of the TRPM8 channel occurs in the presence of certain tumour cells including prostate cancer cell carcinomas and other non-prostatic primary human tumours such as breast, colon, lung and skin cancer.

The subset of TRP channels known as ankyrin-like (TRPA) channels, in particular the TRPA1 channels, are cold-activated channels. The TRPA1 channels have a lower activation temperature in comparison to the TRPM8 channel. The TRPA1 (also known as ANKTM1) channel shares very little amino acid homology with the TRPM8 channel, and as such is thought to be a distant family member of the TRP channels.

The TRPA1 channels have been found to be activated by noxious cold and pungent natural compounds such as those found in cinnamon oil, wintergreen oil, clove oil, mustard oil, raw garlic, camphor and ginger. Bradykinin, which is an inflammatory peptide that acts through the G protein-coupled receptor, is also shown to activate TRPA1.

The topical application of compounds such as mustard oil (allyl isothiocyanate) activates sensory nerve endings; this in turn produces pain, inflammation and a hypersensitivity to both thermal and mechanical stimuli. These effects are caused by activation of TRPA1 channels. Cinnamon oil (cinnamaldehyde) has been shown to be the most specific TRPA1 activator. It excites the TRPA1 channel and is able to elicit nociceptive behaviour in mice. Activation of TRPA1 produces a painful sensation and therefore the elicitation of nociceptive behaviour in mammals provides a model for why noxious cold can be perceived as burning pain, (Bandell et al. Neuron 2004).

The cannabinoid tetrahydrocannabinol (THC) has also been shown to activate the TRPA1 channels (Jordt et al. Nature 2004) acting in a similar manner to mustard oil and cinnamaldehyde.

TRPA1 is also targeted by environmental irritants, such as acrolein, which account for toxic and inflammatory actions of tear gas, vehicle exhaust, and metabolic byproducts of chemotherapeutic agents.

The use of TRPA1-deficient mice has shown that this channel is the sole target through which mustard oil and garlic activate primary afferent nociceptors to produce inflammatory pain. The TRPA1-deficient mice display normal cold sensitivity and unimpaired auditory function, suggesting that this channel is not required for the initial detection of noxious cold or sound. However, these mice exhibit pronounced deficits in bradykinin-evoked nociceptor excitation and pain hypersensitivity. It can therefore be concluded that TRPA1 is an important component of the transduction machinery through which environmental irritants and endogenous pro-analgesic agents depolarize nociceptors to elicit inflammatory pain (Bautista et al. Cell 2006).

Cold hyperalgesia is an enhanced sensitivity to pain and is a well-documented symptom of inflammatory and neuropathic pain; however, the underlying mechanisms of this condition are poorly understood. It has been found that the pharmacological blockade of TRPA1 in primary sensory neurons is able to reverse cold hyperalgesia that has been caused by inflammation and nerve injury. Therefore blocking TRPA1 in sensory neurons might provide a fruitful strategy for treating cold hyperalgesia caused by inflammation and nerve damage, (Obata et al. J Clin Invest 2005).

Intracellular Ca²⁺ activates human TRPA1 via an EF-hand domain and cold sensitivity occurs indirectly (and non physiologically) through increased [Ca²⁺] during cooling in heterologous systems. (Zurborg et al. Nature Neurosci 2007).

The incidence of cold hyperalgesia following L5 spinal nerve ligation (SNL) has been examined, because it is likely that the activation of two distinct populations of TRPA1- and TRPM8-expressing small neurons underlie the sensation of cold. In the nearby uninjured L4 (dorsal route ganglion (DRG), TRPA1 mRNA expression increased in trkA-expressing small-to-medium diameter neurons from the 1st to 14th day after the L5 SNL. This upregulation corresponded well with the development and maintenance of nerve injury-induced cold hyperalgesia of the hind paw. In contrast, there was no change in the expression of the TRPM8 mRNA/protein in the L4 DRG throughout the 2-week time course of the experiment. In the injured L5 DRG, on the other hand, both TRPA1 and TRPM8 expression decreased over 2 weeks after ligation. Furthermore, intrathecal administration of TRPA1, but not TRPM8, antisense oligodeoxynucleotide suppressed the L5 SNL-induced cold hyperalgesia. Increased TRPA1 in uninjured primary afferent neurons may contribute to the exaggerated response to cold observed in the neuropathic pain model, (Katsura et al. Exp Neurol 2006).

Neuropathic pain is a chronic pain that usually is accompanied or caused by tissue injury. With neuropathic pain, the nerve fibres are often damaged, dysfunctional or injured. These damaged nerve fibres send incorrect signals to other pain centres resulting in the chronic pain. The impact of nerve fibre injury includes a change in nerve function both at the site of injury and areas around the injury.

One example of neuropathic pain is called phantom limb syndrome. This occurs when an arm or a leg has been removed because of illness or injury, but the brain still gets pain messages from the nerves that originally carried impulses from the missing limb. These nerves now misfire and cause pain.

Neuropathic pain often seems to have no obvious cause; but, some common causes of neuropathic pain include: multiple sclerosis, diabetes, back injury, amputation, spinal surgery, HIV infection, shingles, alcoholism and facial nerve problems.

The symptoms of neuropathic pain include shooting and burning pain, tingling and numbness and increased sensitivity to touch or cold.

Current treatments include the use of non-steroidal anti-inflammatory drugs and stronger analgesics such as morphine-based drugs. Anti-convulsant and antidepressant drugs are also often used to treat neuropathic pain.

Very often neuropathic pain can be difficult to treat; in this case a pain specialist may use invasive or implantable device therapies to effectively manage the pain. Electrical stimulation of the nerves involved in neuropathic pain generation may significantly control the pain symptoms.

Unfortunately, neuropathic pain often responds poorly to standard pain treatments and occasionally may get worse instead of better over time. For some people, it can lead to serious disability.

Inflammation is the immune systems first response to infection or irritation. Inflammation often causes redness, swelling, pain and dysfunction of the affected area.

Inflammation may also be associated with other symptoms including: fever, chills, fatigue, loss of energy, headaches, loss of appetite and muscle stiffness.

Inflammation is caused by chemicals from white blood cells being released into the blood or affected tissues in an attempt to rid the body of foreign substances. This release of chemicals increases the blood flow to the area and may result in redness and warmth. Some of the chemicals cause leakage of fluid into the tissues, resulting in swelling. The inflammatory process may stimulate nerves and cause pain.

Inflammation of the joints can also occur, this is caused by an increased number of cells and inflammatory substances within the joint causing irritation, wearing down of cartilage (cushions at the end of bones) and swelling of the joint lining.

Inflammation can also affect organs as part of an autoimmune disorder. For example: Inflammation of the heart (myocarditis), which may cause shortness of breath or leg swelling; Inflammation of the small tubes that transport air to the lungs, which may cause an asthma attack; Inflammation of the kidneys (nephritis), which may cause high blood pressure or kidney failure; and Inflammation of the large intestine (colitis) may cause cramps and diarrhoea.

Pain may not be a primary symptom of the inflammatory disease, since many organs do not have many pain-sensitive nerves. Treatment of organ inflammation is directed at the cause of inflammation whenever possible.

There are a number of treatment options for inflammatory diseases including medications, rest and exercise, and surgery to correct joint damage. The type of treatment prescribed will depend on several factors including the type of disease, the person's age, type of medications he or she is taking, overall health, and medical history and severity of symptoms.

There are many medications available to decrease joint pain, swelling and inflammation and prevent or minimize the progression of the inflammatory disease. The medications include: Non-steroidal anti-inflammatory drugs, corticosteroids and anti-malarial medications.

Blockade of the TRPA1 channel has been shown to relieve cold hyperalgesia. This is an enhanced sensitivity to pain, and is a well-documented symptom of inflammatory and neuropathic pain and as such it is thought that agents that are able to blockade the TRPA1 channels could be useful treatments for neuropathic pain and inflammation.

Blockade of the TRPA1 channels also results in vasodilation, therefore agents that are able to produce such an effect might also be useful as vasodilators. Vasodilators are often used to treat conditions such as hypotension or blood clots when there is a requirement to dilate the blood vessels.

Cannabinoids are a group of chemicals known to activate cannabinoid receptors in cells. These chemicals, which are found in cannabis plants, are also produced endogenously in humans and other animals, these are termed endocannabinoids. Synthetic cannabinoids are chemicals with similar structures to plant cannabinoids or endocannabinoids.

Plant cannabinoids can also be isolated such that they are "essentially pure" compounds. These isolated cannabinoids are essentially free of the other naturally occurring compounds, such as, other minor cannabinoids and molecules such as terpenes. Essentially pure compounds have a degree of purity up to at least 95% by total weight.

The cannabinoid tetrahydrocannabinol (THC) has been shown to activate the TRPA1 channels (Jordt et al. Nature 2004) by acting in a similar manner to mustard oil and cinnamaldehyde. The type of THC used in this study was synthetic THC. Synthetic THC such as dronabinol can cause many side effects in users. Such side effects include: palpitations, tachycardia, facial flush, abdominal pain, nausea, vomiting, amnesia, anxiety/nervousness, ataxia, confusion, depersonalization, dizziness, euphoria, hallucination, paranoia, somnolence, hypotension, diarrhoea, depression, nightmares and vision difficulties.

Surprisingly the applicants have found that the administration of cannabinoid-containing plant extracts, are efficacious in the blockade of TRPV1, TRPM8 and TRPA1 channels. In particular cannabinoid-containing plant extracts comprising as a predominant cannabinoid either tetrahydrocannabinol (THC), tetrahydrocannabinolic acid (THCA), cannabidiol (CBD), cannabidiolic acid (CBDA), cannabigerol (CBG) or cannabichromene (CBC) were particularly efficacious.

The term "cannabinoid-containing plant extract" is taken herein to refer to one or more plant extracts from the cannabis plant. A cannabinoid-containing plant extract contains in addition to one or more other cannabinoids, one or more non-cannabinoid components which are co-extracted with the cannabinoids from the plant material. The degree of purity obtained and the respective ranges of additional cannabinoids in the cannabinoid-containing plant extract will vary according to the starting plant material and the extraction methodology used.

Cannabinoid-containing plant extracts may be obtained by various means of extraction of cannabis plant material. Such means include but are not limited to: supercritical or subcritical extraction with CO₂, extraction with hot gas and extraction with solvents.

### SUMMARY OF INVENTION

According to the first aspect of the present invention there is provided one or more cannabinoids, which are TRPM8 antagonists, selected from the group consisting of cannabidiol (CBD); cannabigerol (CBG); and cannabidiolic acid (CBDA) for use in the prevention or treatment of cancer of the prostate, where TRPM8 activity is essential for the cancers survival.

The cannabinoids may be extracted from a cannabis plant using the subcritical CO₂ extraction technique as described in the applicants granted United Kingdom patent GB2391865.

Another cannabis plant extraction technique is extraction with hot gas as described in the applicants granted United Kingdom patent GB2376464.

Preferably the cannabinoids are present as a cannabis based medicine extract (CBME).

A CBME is a plant extract from the cannabis plant and as such depending on the extraction technique used will comprise all of the "naturally extracted" cannabis plant components.

Alternatively the cannabinoid-containing plant extract is isolated or substantially pure.

Isolated or substantially pure cannabinoids will be substantially free of other non-target cannabinoids and other non-cannabinoid components such as terpenes. The isolated or substantially pure cannabinoids may be of natural i.e. plant origin or they may be synthetically produced compounds.

The process disclosed in the applicants granted United Kingdom patent GB2393721 describes a process for preparing substantially pure cannabinoids.

"Substantially pure" is defined herein as preparations of cannabinoid compounds or derivatives thereof having a chromatographic purity of greater than 95%, preferably greater than 96%, more preferably greater than 97%, more preferably greater than 98%, more preferably greater than 99% and most preferably greater than 99.5%, as determined by area normalisation of an HPLC profile.

In one embodiment the cannabinoid-containing plant extract is packaged for delivery in a titratable dosage form.

The term "titrate" is defined as meaning that the patient is provided with a medication that is in such a form that smaller doses than the unit dose can be taken.

A "unit dose" is herein defined as a maximum dose of medication that can be taken at any one time or within a specified dosage period such as 3 hours.

Titration of doses is beneficial to the patient as they are able to increase the dose incrementally until the drug is efficacious. It is understandable that not all patients will require exactly the same dose of medication, for example patients of a larger build or faster metabolism may require a higher dose than that required by a patient that is of a smaller build. Different patients may also present with different degrees of complaints and as such may require larger or smaller doses in order to treat the complaint effectively. The benefits of a titratable dosage form over a standard dosage form, which would have to be split into a partial dose, are therefore evident.

Unit dose ranges for the cannabinoid-containing plant extract may be determined by reference to the cannabinoid content which is preferably in the range of between 5 and 100mg of the total cannabinoids.

Preferably the pharmaceutical formulations are packaged for delivery such that delivery is targeted to an area selected from one or more of the following: sublingual; buccal; oral; rectal; nasal; parenteral and via the pulmonary system.

More preferably the pharmaceutical formulations are in the form selected from one or more of the following: gel; gel spray; tablet; liquid; capsule, by injection and for vaporisation.

Additionally the pharmaceutical formulation further comprises one or more carrier solvents. Preferably the carrier solvents are ethanol and/or propylene glycol. More preferably the ratio of ethanol to propylene glycol is between 4:1 and 1:4. More preferably still the ratio is substantially 1:1.

As discussed in further detail herein, by "treatment" is meant at least improvement, preferably cure of the condition in question.

Certain aspects of this invention are further described, by way of example only.

### SPECIFIC DESCRIPTION

The applicants have conducted experiments using cannabinoid-containing plant extracts on rat recombinant TRPV1, TRPM8 channels and TRPA1 (also known as ANKTM1) channels, both were stably expressed in HEK293 cells. The TRPA1 channel is the receptor for mustard oil isothiocyanates and other plant natural products such as cinnamaldehyde. The TRPM8 channel is the receptor fro menthol and icilin. The intracellular Ca²⁺ concentration was determined before and after the addition of various concentrations of test compounds. Data presented from experiments on the TRPV1 and TRPA1 channels are not the subject of the invention and are included for information only.

Surprisingly it was discovered that the cannabinoid-containing plant extracts were able to blockade the channels tested and produced estimated EC₅₀ values in the 100 nM range, and below.

### Example 1:

### The effects of cannabinoid-containing plant extracts on intracellular Ca²⁺ concentration

### Materials and methods

### Compounds

For the experiments with the TRPA1 channels allyl isothiocyanate (mustard oil) and cinnamaldehyde were used as positive controls to which the values obtained from cannabinoid-containing plant extracts were compared to. For the experiments with the TRPM8 channels menthol and icilin were used to activate the channels. For the experiments with the TRPV1 channels ionomycin was used to activate the channels. The cannabinoid-containing plant extracts were produced from cannabis plants using the subcritical CO₂ extraction technique as described in the applicants granted United Kingdom patent GB2391865. The cannabinoids were then purified further using the method disclosed in the applicants granted United Kingdom patent GB2393721 to produce substantially pure cannabinoids. Unpurified plant extracts of THC and CBD were also tested in the experiments with the TRPA1 channels.

### Permanent transfection of HEK-293 cells with rat TRPA1, TRPV1 or TRPM8 cDNA

HEK293 (human embryonic kidney) cells were plated on 100 mm diameter Petri dishes and transfected at about 80% confluence with Lipofectamine 2000 (Invitrogen) using a plasmid containing the rat TRPA1, TRPV1 or TRPM8 cDNA, according to the manufacturer's protocol. A stably transfected clone was selected by Geneticin G-418 (Invitrogen) 600 µg/ml. Stable transfection was checked by quantitative real time-PCR (RT-PCR). PCR analysis on the DNA from TRPA1/TRPV1/TRPM8-HEK293 cells demonstrated full integration of gene into HEK293 cell genome (not shown).

### Experiments in HEK-293 cells over-expressing the rat TRPA1, TRPV1 or TRPM8 channel

TRPA/TRPV1/TRPM8-HEK-293 cells were plated on 100 mm diameter Petri dishes and after 3 days loaded for 1 hour at room temperature with the cytoplasmic calcium indicator Fluo4-AM (4 µM, Molecular Probes) dissolved in Tyrode' buffer (NaCl 145 mM; KCl 2.5 mM; CaCl₂ 1.5 mM; MgCl₂ 1.2 mM; D-Glucose 10 mM; HEPES 10 mM pH 7.4) containing Pluronic (0.02%, Molecular Probes). The cells were washed twice in Tyrode buffer, resuspended and transferred to the quartz cuvette of the spectrofluorimeter (Perkin -Elmer LS 50B) (λ excitation = 488 nm; λ emission = 516 nm). Intracellular Ca²⁺ concentration was determined before and after the addition of various concentrations of test compounds. ECso values were determined as the concentration of test substances required to produce half-maximal increases in intracellular Ca²⁺ concentration. Curve fitting and parameter estimation was performed with Graph Pad Prism®. The same compounds were tested also on non-transfected HEK 293 cells.

### Results

As can be observed by looking at the values in Tables 1 and 2 derived from the experiments, the log EC50% values for the cannabinoids tested demonstrate that the cannabinoids tested were able to produce a blockade of the TRP channels.

**Table 1: TRPA1 channel**

| Test substance | **-logEC_{50%} (M) for the elevation of [Ca²⁺] ᵢ** | **Maximal response (% of mustard oil 100 µM)** |
|---|---|---|
| Mustard oil (allyl isothiocyanate) | 5.60 ± 0.15 | 100±11 |
| Cinnamaldehyde | 4.89 ± 0.17 | 99.1±9 |
| THC | 6.73 ± 0.18 | 116.9±12 |
| THCA | 6.85 ± 0.27 | 70.1±8 |
| CBD | 7.07 ± 0.03 | 86.9±8 |
| CBDA | 6.09 ± 0.02 | 48.2±6 |
| CBC | 7.48 ± 0.31 | 117.5±10 |
| CBD plant extract | 6.28 ± 0.21 | 80.5±7 |
| THC plant extract | 7.93 ± 1.90 | 79.5±8 |

The values obtained for the cannabinoids were highly comparable to that of mustard oil and cinnamaldehyde, and their potency can be ranked as follows: THC extract > CBC > CBD > THC > THCA > CBD extract > CBDA > mustard oil > cinnamaldehyde. In particular, THC extract, CBC and CBD exhibited EC₅₀ values in the 60-100 nM range of concentrations. These data suggest that TRPA1 might be one of the molecular targets underlying some of the pharmacological actions of phytocannabinoids.

These data are significant, as at the present time there are few useful treatment options for patients suffering form neuropathic pain, inflammation or vasoconstriction and the use of cannabinoids in the production of a pharmaceutical formulation that could be used to treat such conditions would prove useful.

**Table 2: TRPM8 channel blockade**

| | pIC₅₀ *vs*. menthol 50 µM | pIC₅₀ *vs.* icilin 0.25 µM |
|---|---|---|
| CBC | <5 | <5 |
| THC | 6.86 ± 0.04 | 6.85 ± 0.08 |
| THCA | 7.17 ± 0.05 | 6.92 ± 0.08 |
| CBD | 6.89 ± 0.11 | 7.02 ± 0.05 |
| CBDA | 5.84 ± 0.17 | 5.99 ± 0.06 |
| CBG | 6.79 ± 0.09 | 6.84 ± 0.02 |

The table above details the potency of the cannabinoids at blockade of the TRPM8 channel. The values obtained demonstrate that the cannabinoids were all effective at blockade and as such TRPM8 antagonists might provide new therapeutic tools for the treatment of cancers where TRPM8 activity is essential the cancer cells survival.

It has previously been shown that cannabinoid-containing plant extracts can be used either alone or in combination to usefully treat various diseases and conditions. These data presented herein provide evidence for the use of cannabinoid-containing plant extracts for the treatment of diseases and conditions that are alleviated by blockade of the TRP channels. Herein it has been demonstrated that all of the cannabinoids tested produced an activation of the TRPA1 channels and as such could be useful in the prevention or treatment of diseases or conditions that are alleviated by activation of the TRPA1 channels. It has also been demonstrated that the cannabinoids tested are able to antagonize the TRPM8 channels and as such are potentially of use in the prevention or treatment of diseases or conditions that are alleviated by antagonism of the TRPM8 channels.

**Table 3: TRPV1 channel blockade**

| Test substance | **EC_{50%} (M) for the elevation of [Ca²⁺] ᵢ** | **Maximal response (% ionomycin)** |
|---|---|---|
| Capsaicin | 10 nM | 68.6±1.2 |
| CBC-BDS | 11.9 µM (CBC equivalent) | 35.2±1.0 |
| CBG-BDS | 4.6 µM (CBG equivalent) | 32.5±3.4 |
| CBC | 24.2 µM | 9.0±4.9 |
| CBD | 0.7 µM | 50.0±1.0 |
| CBDV | 1.4 µM | 19.8±1.9 |
| CBG | 1.0 µM | 54.4±5.4 |

The table above details the potency of the cannabinoids at blockade of the TRPV1 channel. The values obtained demonstrate that the cannabinoids were all effective at blockade and as such TRPV1 antagonists might provide new therapeutic tools for the treatment of patients suffering from neuropathic pain, inflammation or vasoconstriction. As can be observed by looking at the values in Tables 1 and 2 derived from the experiments, the log EC50% values for the cannabinoids tested demonstrate that the cannabinoids tested were able to produce a blockade of the TRP channels.

**Table 1: TRPA1 channel**

| Test substance | **-logEC_{50%} (M) for the elevation of [Ca²⁺] ᵢ** | **Maximal response (% of mustard oil 100 µM)** |
|---|---|---|
| Mustard oil (allyl isothiocyanate) | 5.60 ± 0.15 | 100±11 |
| Cinnamaldehyde | 4.89 ± 0.17 | 99.1±9 |
| THC | 6.73 ± 0.18 | 116.9±12 |
| THCA | 6.85 ± 0.27 | 70.1±8 |
| CBD | 7.07 ± 0.03 | 86.9±8 |
| CBDA | 6.09 ± 0.02 | 48.2±6 |
| CBC | 7.48 ± 0.31 | 117.5±10 |
| CBD plant extract | 6.28 ± 0.21 | 80.5±7 |
| THC plant extract | 7.93 ± 1.90 | 79.5±8 |

The values obtained for the cannabinoids were highly comparable to that of mustard oil and cinnamaldehyde, and their potency can be ranked as follows: THC extract > CBC > CBD > THC > THCA > CBD extract > CBDA > mustard oil > cinnamaldehyde. In particular, THC extract, CBC and CBD exhibited EC₅₀ values in the 60-100 nM range of concentrations. These data suggest that TRPA1 might be one of the molecular targets underlying some of the pharmacological actions of phytocannabinoids.

These data are significant, as at the present time there are few useful treatment options for patients suffering form neuropathic pain, inflammation or vasoconstriction and the use of cannabinoids in the production of a pharmaceutical formulation that could be used to treat such conditions would prove useful.

**Table 2: TRPM8 channel blockade**

| | pIC₅₀ *vs.* menthol 50 µM | pIC₅₀ *vs*. icilin 0.25 µM |
|---|---|---|
| CBC | <5 | <5 |
| THC | 6.86 ± 0.04 | 6.85 ± 0.08 |
| THCA | 7.17 ± 0.05 | 6.92 ± 0.08 |
| CBD | 6.89 ± 0.11 | 7.02 ± 0.05 |
| CBDA | 5.84 ± 0.17 | 5.99 ± 0.06 |
| CBG | 6.79 ± 0.09 | 6.84 ± 0.02 |

The table above details the potency of the cannabinoids at blockade of the TRPM8 channel. The values obtained demonstrate that the cannabinoids were all effective at blockade and as such TRPM8 antagonists might provide new therapeutic tools for the treatment of cancers where TRPM8 activity is essential the cancer cells survival.

It has previously been shown that cannabinoid-containing plant extracts can be used either alone or in combination to usefully treat various diseases and conditions. These data presented herein provide evidence for the use of cannabinoid-containing plant extracts for the treatment of diseases and conditions that are alleviated by blockade of the TRP channels. Herein it has been demonstrated that all of the cannabinoids tested produced an activation of the TRPA1 channels and as such could be useful in the prevention or treatment of diseases or conditions that are alleviated by activation of the TRPA1 channels. It has also been demonstrated that the cannabinoids tested are able to antagonize the TRPM8 channels and as such are potentially of use in the prevention or treatment of diseases or conditions that are alleviated by antagonism of the TRPM8 channels.

**Table 3: TRPV1 channel blockade (illustrative)**

| Test substance | **EC_{50%} (M) for the elevation of [Ca²⁺] ᵢ** | **Maximal response (% ionomycin)** |
|---|---|---|
| Capsaicin | 10 nM | 68.6±1.2 |
| CBC-BDS | 11.9 µM (CBC equivalent) | 35.2±1.0 |
| CBG-BDS | 4.6 µM (CBG equivalent) | 32.5±3.4 |
| CBC | 24.2 µM | 9.0±4.9 |
| CBD | 0.7 µM | 50.0±1.0 |
| CBDV | 1.4 µM | 19.8±1.9 |
| CBG | 1.0 µM | 54.4±5.4 |

The table above details the potency of the cannabinoids at blockade of the TRPV1 channel. The values obtained demonstrate that the cannabinoids were all effective at blockade and as such TRPV1 antagonists might provide new therapeutic tools for the treatment of patients suffering from neuropathic pain, inflammation or vasoconstriction.

## Claims

1. One or more cannabinoids, which are TRPM8 antagonists, selected from the group consisting of cannabidiol (CBD), cannabigerol (CBG); and cannabidiolic acid (CBDA) for use in the prevention or treatment of cancer of the prostate, where TRPM8 activity is essential for the cancer cells survival.

2. Cannabinoids for the use as claimed in claim 1, wherein the cannabinoid is cannabidiol (CBD).

3. Cannabinoids for the use as claimed in claim 1, wherein the cannabinoid is cannabidiolic acid (CBDA).

4. Cannabinoids for the use as claimed in claim 1, wherein the cannabinoid is cannabigerol (CBG) as a predominant cannabinoid.

5. Cannabinoids for the use as claimed in any of the preceding claims, wherein the cannabinoids are present as a cannabis based medicine extract (CBME).

6. Cannabinoids for the as claimed in claim 5, wherein the one or more cannabis based medicine extract (CBME) comprises all of the naturally extracted cannabis plant components.

7. Cannabinoids for the use as claimed in claim 1, wherein the cannabinoids are natural or synthetically produced.

8. Cannabinoids for the use as claimed in claim 1, wherein the cannabinoids are isolated or substantially pure.

9. Cannabinoids for the use as claimed in any of the preceding claims, wherein the cannabinoids are packaged for delivery in a titratable dosage form.

## Patentansprüche

1. Ein oder mehrere Cannabinoide, die TRPM8 Antagonisten sind, ausgewählt aus der Gruppe bestehend aus Cannabidiol (CBD), Cannabigerol (CBG) und Cannabidiolsäure (CBDA) zur Verwendung in der Prävention oder Behandlung von Prostatakrebs, wobei TRPM8-Aktivität für das Überleben der Krebszellen wesentlich ist.

2. Cannabinoide zur Verwendung nach Anspruch 1, wobei das Cannabinoid Cannabidiol (CBD) ist.

3. Cannabinoide zur Verwendung nach Anspruch 1, wobei das Cannabinoid Cannabidiolsäure (CBDA) ist.

4. Cannabinoide zur Verwendung nach Anspruch 1, wobei das Cannabinoid Cannabigerol (CBG) als überwiegendes Cannabinoid ist.

5. Cannabinoide zur Verwendung nach einem der vorstehenden Ansprüche, wobei die Cannabinoide als Arzneimittelextrakt auf Cannabis-Basis (CBME) vorhanden sind.

6. Cannabinoide zur Verwendung nach Anspruch 5, wobei das eine oder die mehreren Arzneimittelextrakte auf Cannabis-Basis (CBME) alle natürlich extrahierten Bestandteile der Cannabispflanze umfassen.

7. Cannabinoide zur Verwendung nach Anspruch 1, wobei die Cannabinoide natürlich oder synthetisch erzeugt sind.

8. Cannabinoide zur Verwendung nach Anspruch 1, wobei die Cannabinoide isoliert oder im Wesentlichen rein sind.

9. Cannabinoide zur Verwendung nach einem der vorstehenden Ansprüche, wobei die Cannabinoide für eine Verabreichung in einer titrierbaren Darreichungsform verpackt sind.

## Revendications

1. Un ou plusieurs cannabinoïdes, qui sont des antagonistes du TRPM8, choisis dans le groupe constitué par le cannabidiol (CBD), le cannabigérol (CBG) et l'acide cannabidiolique (CBDA), destinés à être utilisés dans la prévention ou le traitement du cancer de la prostate, l'activité du TRPM8 étant essentielle à la survie des cellules cancéreuses.

2. Cannabinoïdes destinés à être utilisés selon la revendication 1, le cannabinoïde étant du cannabidiol.

3. Cannabinoïdes destinés à être utilisés selon la revendication 1, le cannabinoïde étant de l'acide cannabidiolique (CBDA).

4. Cannabinoïdes destinés à être utilisés selon la revendication 1, le cannabinoïde étant du cannabigérol (CBG) comme cannabinoïde prédominant.

5. Cannabinoïdes destinés à être utilisés selon l'une quelconque des revendications précédentes, les cannabinoïdes étant présents en tant qu'un extrait de médicament à base de cannabis (CBME).

6. Cannabinoïdes destinés à être utilisés selon la revendication 5, l'extrait de médicament à base de cannabis (CBME) comprenant tous les composants végétaux de cannabis extraits naturellement.

7. Cannabinoïdes destinés à être utilisés selon la revendication 1, les cannabinoïdes étant produits naturellement ou synthétiquement.

8. Cannabinoïdes destinés à être utilisés selon la revendication 1, les cannabinoïdes étant isolés ou sensiblement purs.

9. Cannabinoïdes destinés à être utilisés selon l'une quelconque des revendications précédentes, les cannabinoïdes étant conditionnés pour être administrés sous une forme dosifiée titrable.
